(19) Europäisches Patentamt
European Patent Office
Office européen des brevets

(11) Publication number : 0 366 212 B1

(12) **EUROPEAN PATENT SPECIFICATION**

(45) Date of publication of patent specification : **20.01.93 Bulletin 93/03**

(51) Int. Cl.$^5$ : **C07C 2/34,** B01J 31/22

(21) Application number : **89202697.2**

(22) Date of filing : **25.10.89**

# (54) Ethylene oligomerization process and catalyst.

(30) Priority : **27.10.88 US 263449**
**08.08.89 US 389476**

(43) Date of publication of application :
**02.05.90 Bulletin 90/18**

(45) Publication of the grant of the patent :
**20.01.93 Bulletin 93/03**

(84) Designated Contracting States :
**BE DE FR GB IT NL**

(56) References cited :
**EP-A- 0 069 951**
**EP-A- 0 257 696**
**EP-A- 0 268 214**
**DE-A- 3 731 107**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Slaugh, Lynn Henry**
**12638 Rifleman Trail**
**Cypress Texas 77429 (US)**
Inventor : **Schoenthal, Galeon Wayne**
**403 N. Foothill Blvd.**
**Cloverdale California 95425 (US)**

EP 0 366 212 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to a process for oligomerizing ethylene and a zirconocene/aluminoxane catalyst suitable for use in the process.

Oligomers of ethylene and oligomers of ethylene and lower molecular weight alpha olefins are useful as intermediates in the preparation of lubricant additives or speciality detergents. Typically, oligomers of ethylene having from 4 to 8 carbon atoms are useful for the preparation of lubricant additives, and oligomers of ethylene having from 10 to 20 carbon atoms are useful for the preparation of detergents. Accordingly, there is a need for processes for preparing oligomers of ethylene having from 4 to 20 carbon atoms.

It is well known in the art that olefins may be catalytically converted into dimers, higher oligomers and polymers.

European patent application publication No. 0069951 (corresponding to United States patent No. 4,542,199) discloses that polymers of olefins such as ethylene may be prepared using a catalyst comprising a metallocene such as a zirconocene and either a methylaluminoxane or an ethylaluminoxane. The atomic ratio of aluminium to metal employed in the Examples is very high, typically about 100,000:1.

European patent application publication No. 0257696 (corresponding to United States patent No. 4,658,078) discloses that dimers of olefins of general formula

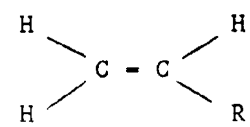

wherein R represents a $C_{1-30}$ alkyl, cycloalkyl or cycloalkenyl group (i.e. excluding ethylene) may be prepared using a catalyst comprising a zirconocene or hafnocene and an aluminoxane wherein the atomic ratio of zirconium or hafnium to aluminium is in the range of from 1 to 100. The aluminoxane is a $C_{1-5}$ alkylaluminoxane, preferably a methylaluminoxane.

European patent application publication No. 0226463 (corresponding to International patent application No. U.S. 86/02667) discloses that polymers of ethylene may be prepared using a catalyst comprising a metallocene such as a zirconocene and an excess of an aluminoxane such as methylaluminoxane. The atomic ratio of aluminium to metal is preferably in the range of from 12 to 100:1.

European patent application publication No. 0268214 discloses that oligomers can be prepared from propylene using a catalyst comprising a metallocene such as zirconocene and a condensation product of an organoaluminium compound and water. The organoaluminium compound can be at least one of methylaluminium or triethylaluminium. However, no further information is given about the implicitly disclosed mixtures of both, and the examples disclose only methylaluminium.

Surprisingly, it has now been found that oligomers of ethylene and oligomers of ethylene with lower molecular weight alpha olefins may advantageously be prepared using a catalyst comprising a particular mixed methyl/ethyl aluminoxane.

Accordingly, the present invention provides a process for preparing oligomers of ethylene, which comprises contacting ethylene, alone or in combination with one or more $C_3$-$C_{10}$ alpha olefins, at an elevated temperature with a catalyst comprising the reaction product of a zirconocene and one or more alkyl aluminoxanes wherein the alkyl is selected from methyl, ethyl and mixtures thereof and the ratio of equivalents of methyl to ethyl in the product is in the range of from 4 to 0.25 and wherein the atomic ratio of aluminium to zirconium is in the range of from 0.1 to 100.

When ethylene is used alone, the major proportion of the product olefins are alpha olefins. The process has been found to be particularly suitable for converting ethylene to additive range ($C_4$-$C_8$) and detergent range ($C_{10}$-$C_{20}$) olefins.

When ethylene is used in combination with one or more $C_3$-$C_{10}$ alpha olefins, that is when the additional olefin is represented by $RCH = CH_2$ wherein R is alkyl of up to 8 carbon atoms, the product olefins contain significant portions of vinylidene olefins.

The oligomerization reaction may be carried out in a conventional fashion. It may be carried out continuously in a stirred tank reactor wherein olefin and catalyst or catalyst precursors are added continuously to a stirred tank and reactant, product and catalyst and unused reactant are removed from the stirred tank with the product separated and the catalyst and unused reactant recycled back to the stirred tank. Alternatively, the reaction may be carried out in a batch reactor, wherein the catalyst, or the catalyst precursors, and reactant

olefin are charged to an autoclave, and after being reacted for an appropriate time, product is separated from the reaction mixture by conventional means, such as distillation. The reaction may most conveniently be conducted over a range of temperatures from 100 °C to 130 °C, preferably in the range of from 110 °C to 125 °C, more preferably in the range of from 115 °C to 120 °C. Pressures are not critical and are conveniently in the range of from 1 to 507 bar (500 atmospheres) or higher. The oligomerization reaction can be carried out in the gas phase or liquid phase or mixed gas-liquid phase, depending on the volatility of the feed and product olefins.

The oligomerization may be carried out in the presence of an inert solvent which also may be the carrier for the catalyst and/or feed olefin. Suitable solvents include hydrocarbons, such as alkanes and aromatics, for example benzene, xylene and toluene.

The catalyst used in the process according to the invention has a certain adverse sensitivity to oxygen. It is thus desired to carry out the catalyst preparation and oligomerization reaction in the absence of oxygen.

The selectivity of the process according to the invention is believed to be attributable to the particular catalyst employed. This catalyst is believed to be novel, and accordingly forms a further aspect of the invention.

Accordingly, the present invention also provides an olefin oligomerization catalyst comprising the reaction product of a zirconocene and one or more alkyl aluminoxanes wherein the alkyl is both methyl and ethyl and the ratio of equivalents of methyl to ethyl in the product is in the range of from 4 to 0.25 and wherein the atomic ratio of aluminium to zirconium is in the range of from 0.1 to 100.

The zirconocenes employed in the production of the catalyst organometailic compounds which are cyclopentadienyl derivatives of zirconium, and include mono-, di- and tricyclopentadienyl derivatives of zirconium. A preferred zirconocene has the general formula

$$(\text{cyclopentadienyl})_n\text{Zr}\ Y_{4-n}$$

wherein n is an integer ranging from 1 to 4, Y is selected from the group consisting of hydrogen, a $C_1$-$C_5$ alkyl group, a $C_6$-$C_{20}$ aryl group, or halogen, and the cyclopentadienyl groups are optionally substituted by one or more $C_1$-$_5$ alkyl groups.

Examples of the suitable zirconocenes are bis(cyclopentadienyl)zirconium dichloride, bis(cyclopentadienyl)zirconium methyl chloride, bis(cyclopentadienyl)-zirconium dimethyl, bis(methylcyclopentadienyl)zirconium dichloride, bis(methylcyclopentadienyl)zirconium methyl chloride, bis(methylcyclopentadienyl)zirconium dimethyl, bis(pentamethylcyclopentadienyl)zirconium dichloride, bis(pentamethylcyclopentadienyl)zirconium methyl chloride, bis(pentamethylcyclopentadienyl)zirconium dimethyl, bis(n-butylcyclopentadienyl)zirconium dichloride, bis(n-butyl-cyclopentadienyl)zirconium methyl chloride, bis(n-butylcyclopentadienyl)zirconium dimethyl.

Aluminoxanes (or alumoxanes) are well known in the art and are polymeric alkyl aluminium compounds which can be represented by the general formula $(R\text{-}Al\text{-}O)_n$, which is a cyclic compound, and $R(R\text{-}Al\text{-}O)_nAlR_2$, which is a linear compound. In the process and catalyst according to the invention, each R in the general formula is independently a methyl or ethyl group and n is an integer from 1 to 40, preferably 1-2. Generally, in the preparation of aluminoxanes from, for example, a trialkyl aluminium such as trimethyl aluminium and water, a mixture of the linear and cyclic compounds are obtained.

Alkyl aluminoxanes can be prepared in various ways. Preferably, they are prepared by contacting water with a solution a of trialkyl aluminium, such as, for example, trimethyl aluminium or triethyl aluminium, in a suitable organic solvent such as benzene or an aliphatic hydrocarbon. Solvents that can be used are well-known and include saturated aliphatic compounds such as butane, pentane, hexane, heptane, octane, isoctane or purified kerosenes; cycloaliphatics such as cyclobutane, cyclopentane, cyclohexane, cycloheptane, methylcyclopentane or dimethylcyclopentane; and aromatic solvents such as benzene, toluene or xylene. The major requirements in the selection of a solvent are that it be liquid at reaction temperatures and pressures, that it does not react with water or the aluminoxanes or interfere with the desired oligomerization reaction. The solvent must be oxygen-free. Hydroxy, ether, carboxyl, keto groups adversely affect aluminoxane production. A particularly suitable solvent is one or more of the olefins to be oligomerized, if the olefin is a liquid. For example, the alkyl aluminium is treated with water in the form of a moist solvent or the alkyl aluminium can be desirably contacted with a hydrated salt such as hydrated copper sulfate or aluminium sulfate.

The aluminoxane can be prepared in the presence of a hydrated copper sulfate. This method comprises treating a dilute solution of a trialkyl aluminium such as trimethyl aluminium in, for example, toluene, with copper sulfate represented by the general formula $CuSO_4.5H_2O$. The ratio of copper sulfate to trialkyl aluminium is desirably about 1 mol of copper sulfate for 5 mol of trialkyl aluminium. The reaction is evidenced by the evolution of an alkane such as methane. The use of ferrous sulfate heptahydrate as a hydrating agent for trialkyl aluminium is described in European patent application publication No. 0226463.

In general, the molar ratio of alkyl aluminium to water will be 1:1 although variations of this ratio can occur without adversely affecting the aluminoxane product; i.e., the Al/water molar ratio can conveniently vary between 0.66:1 to 2:1, preferably between 0.75:1 to 1.25:1. A continuous method for producing aluminoxanes is

given in U.S. patent 3,300,458. Another suitable method involves the use of hydrated aluminium salts as given in U.S. patent 4,544,762. Another suitable method is to use water which has been ultrasonically dispersed in a solvent as described in U.S. patent 4,730,071, or water which has been dispersed using high speed shearing as described in U.S. patent 4,730,072.

A key aspect of this invention is the use of mixed methylethyl aluminoxanes to produce the catalyst. The alkyl aluminoxane(s) used to produce the catalysts of the instant invention comprise one or more alkyl aluminoxanes wherein the alkyl is selected from methyl, ethyl and mixtures thereof and wherein the ratio of methyl groups to ethyl groups in the catalyst product falls within a defined range. The aluminoxane(s) used will have a ratio of methyl groups to ethyl groups in the range of from 4 to 0.25, preferably from 2 to 0.33 and more preferably from 1 to 0.5. The mixed aluminoxanes may be prepared from appropriate amounts of trimethyl aluminium, triethyl aluminium, diethylmethyl aluminium and ethyldimethyl aluminium as needed to provide the required ratio of methyl to ethyl. Trimethyl and triethyl aluminium compounds are more readily available than are the mixed methyl/ethyl aluminium compounds.

There are several substantially equivalent methods that can be used to prepare the mixed aluminoxane. For example, appropriate amounts of trimethyl aluminium and triethyl aluminium can be individually hydrolyzed and the resultant aluminoxanes combined to provide the desired mixture of methyl and ethyl aluminoxane. Also, trimethyl aluminium and triethyl aluminium can be mixed and the resultant mixture hydrolyzed to produce the desired mixture of methyl and ethyl aluminoxane. Methyldiethyl and dimethylethyl aluminium compounds, either alone or suitably mixed with each other or with trimethyl and/or triethyl aluminium can also be utilized.

In general terms the catalyst of the instant invention is prepared by reacting a zirconocene with the aluminoxane(s) in the presence of a suitable solvent. When only one alkyl aluminoxane is used, the alkyl will comprise a mixture of methyl and ethyl groups. When more than one alkyl aluminoxane is used, each alkyl aluminoxane may comprise individually either methyl aluminoxane or ethyl aluminoxane or a mixed methylethyl aluminoxane with the proviso that in the final product the methyl/ethyl ratio will fall within the defined range. The order of addition in contacting the zirconocene and aluminoxane(s) can vary. For example, the metallocene (neat or dissolved in a suitable solvent) can be first added to the reaction vessel followed by the addition thereto of the alumoxane(s); the alumoxane(s) and metallocene can be added to the reaction vessel simultaneously; the alumoxane(s) can be first added to the reaction vessel followed by the addition of the metallocene. Alternatively, a portion of the zirconocene can be reacted, for example, with methyl alumoxane, the remaining portion of the zirconocene reacted with ethyl alumoxane and then both reaction mixtures reacted together to produce the instant compositions. In accordance with the preferred embodiment of this invention the metallocene dissolved in a suitable inert hydrocarbon solvent is added to a stirred solution of the alumoxane(s).

The preparation of the metallocene-alumoxane reaction product, as mentioned above, is preferably conducted in an inert solvent, preferably a hydrocarbon solvent in which the metallocene and alumoxane or the reaction product of the metallocene and alumoxane are soluble. Preferred solutions include mineral oils and the various hydrocarbons which are liquid at reaction temperatures and in which the individual ingredients and/or the product are soluble. Illustrative examples of useful solvents include the alkanes such as pentane, iso-pentane, hexane, heptane, octane, nonane, and the like; cycloalkanes such as cyclopentane, cyclohexane, and the like; and aromatics such as benzene, toluene, ethylbenzene, diethylbenzene, and the like. The amount of solvent to be employed can vary over a wide range without a deletereous effect of the reaction.

At all times, the individual ingredients as well as the recovered catalyst are protected from oxygen and moisture. Therefore, the reactions must be performed in an oxygen and moisture free atmosphere and recovered in an oxygen and moisture free atmosphere. Preferably, therefore, the reaction is performed in the presence of an inert dry gas such as, for example, helium or nitrogen. The recovered catalyst can be maintained in a nitrogen atmosphere, preferably at subambient temperature. The catalyst may also be stabilized by the addition of 3,3,3-trialkyl-1-propene as disclosed in U.S. patent 4,665,047.

The zirconocenes and aluminoxane are reacted to provide an atomic ratio of Al to Zr of from 0.1:1 to 1:100, preferably from 1:1 to 1:50 more preferably from 1:1 to 1:20 and even more preferably from 1:1 to 1:10. Preferred are low ratios of from 1:1 to 1:5. Atomic ratios of Al to Zr of less than 1:1 can be utilized. However, with these lesser limits a certain amount of the zirconocene will be "wasted" as a catalyst since the amount of zirconocene in excess of the equivalent amount of alumoxane will not be combined with the alumoxane and will not serve as a very effective catalyst. Although less effective, these lesser amounts are still considered within the scope of the instant invention. Atomic ratios of Al to Zr of below 0.1 and more preferably below 0.5 are not particularly desired.

The present invention will now be illustrated by means of the following Examples.

The following abbreviations are used: Et for ethyl; Me for methyl; i-Bu for isobutyl; Cp for cyclopentadienyl; g.c. for gas chromatography. Molar amounts of an aluminoxane are determined on the basis of the atomic amounts of aluminium present. For example, one mol of aluminoxane and one mol of the trialkyl aluminium

precursor used to prepare the aluminoxane are considered molar equivalents.

Preparation of Mixed Aluminoxane

32 g of $Al_2(SO_4)_3.18H_2O$ were ground to a powder and placed in a 500 ml round bottomed flask fitted with a stopcock assembly for an inlet and an outlet to a gas bubbler. 50 ml of toluene which had been dried with 3A molecular sieve was added to the flask, which was then purged with nitrogen while being chilled in a wet ice bath. The toluene was then stirred magnetically while 150 ml of 25% triethyl aluminium in n-heptane (calculated to be 0.28 mol of triethyl aluminium) and 50 ml of 25% trimethyl aluminium in toluene (calculated to be 0.14 mol trimethyl aluminium) were added. The flask was maintained in the wet ice bath for the first four hours after addition of the aluminium alkyls, and was then allowed to warm up to ambient temperature. 69 Hours after the addition of the aluminium alkyls, the flask was stoppered under nitrogen and transferred to a dry box. The product was filtered and washed to solid with 25 ml of toluene. Solvent and aluminium alkyl were stripped on a rotary evaporator with gentle warming. 24 g were recovered.

Catalyst Preparation

The catalysts described in the following Examples were prepared in situ as the catalyst ingredients and olefin substrate were warmed to reaction temperature while being mechanically stirred inside a dry box. The solvent, aluminoxane and bis(cyclopentadienyl)zirconium dichloride were placed in a dry, clean 100 ml Parr Autoclave. When a liquid olefin was to be used, it also was placed in the autoclave along with the catalyst components and the solvent. Gaseous olefin substrates, such as ethylene, propylene or 1-butene were introduced into the sealed autoclave under pressure. As the autoclave was heated to reaction temperature and the contents stirred, the catalyst components reacted to form the active catalyst in situ.

Oligomerization Procedure

The following illustrates the typical oligomerization procedures used in the oligomerization of ethylene.

Experiments were conducted with a 100 ml stirred Parr Autoclave. The catalyst and solvent were loaded in a dry box. The autoclave had previously been heated to remove moisture before introduction into the dry box. Solvents were dried over 3A molecular sieve and in the case of hydrocarbons, sodium was added for at least two days before use. After addition of materials to the autoclave, the vessel was weighed outside the dry box, and set up for stirring, heating and introduction of ethylene as quickly as possible. In some cases the autoclave was heated directly to near reaction temperature before addition of the ethylene. In other cases a small amount of ethylene was introduced to the autoclave during heatup. Heatup time was usually near three minutes before ethylene was added to bring the autoclave up to reaction pressure. Stirring was approximately at 700 revolutions per minute (RPM). Ethylene was c.p. grade and no attempt was made for further purification. After the reaction period, ethylene supply was shut off and the reactor was quickly cooled in a wet ice bath. The gas was vented and the reactor was reweighed to measure product weight. Nonane was added as an internal standard for gas chromatographic analysis on a capillary column.

Example 1

A series of ethylene oligomerizations were carried out similar to that described above. The ethylene pressure was 14.8 bar (200 psig). The reaction time was 0.5 hours. 2 mmol each of aluminoxane and $(Cp)_2$ $ZrCl_2$ were used to prepare the catalyst. 40 ml of toluene were used in the autoclave as solvent. The reaction was carried out at the temperature specified in Table 1. The aluminoxane catalyst precursor was prepared either by hydrolyzing a mixture of $Et_3Al$ and $Me_3Al$ or by separately hydrolyzing $Et_3Al$ and $Me_3Al$ and combining separately. The catalyst was prepared in the autoclave by adding the $(Cp)_2ZrCl_2$ and the aluminoxane to the toluene solvent. The particular ethyl to methyl ratio used in the aluminoxane is shown in Table 1. The product was analyzed by g.c. and the results are shown in Table 1.

Table 1

| Example | Aluminoxane Ethyl:Methyl Ratio | Temp., °C | Product Wt. g | Product Distribution $C_4$ | $C_6$ | $C_8$ | $C_{10}$ | $C_{12}$ | $C_{14}$ | $C_{16}$ | $C_{18}$ | $C_{20}^+$ |
|---|---|---|---|---|---|---|---|---|---|---|---|---|
| 1-1 | 2:1[a)] | 116-118 | 12.4 | 13.4 | 16.9 | 14.8 | 8.9 | 7.2 | 5.4 | 4.0 | 2.5 | 27.0 |
| 1-2 | 1:1[b)] | 116-118 | 12.3 | 13.4 | 14.9 | 14.0 | 9.1 | 7.5 | 5.6 | 4.2 | 3.0 | 28.3 |
| 1-3 | 1:1[e)] | 116-118 | 9.8 | 20.2 | 20.3 | 15.0 | 9.0 | 6.7 | 4.9 | 3.5 | 2.1 | 18.3 |
| 1-4 | 2:1[a)] | 105-110 | 24.8 | 13.2 | 4.6 | 6.5 | 5.8 | 6.3 | 5.0 | 4.2 | 3.1 | 51.3 |
| Comparison 1A | 0:1[c)] | 116-120 | 12.9 | 3.7 | 5.3 | 6.2 | 5.8 | 6.0 | 5.8 | 5.6 | 5.1 | 56.5 |
| Comparison 1B | 1:0[d)] | 115-119 | 2.0 | 34.5 | 23.0 | 8.0 | 3.5 | 1.5 | 1.0 | 0.5 | 0 | 28.0 |

a) Aluminoxane prepared by controlled hydrolysis of a 2:1 mixture of $Et_3Al$ and $Me_3Al$ with $Al_2(SO_4)_3 \cdot 18H_2O$.

b) Aluminoxane prepared by controlled hydrolysis of a 1:1 mixture of $Et_3Al$ and $Me_3Al$ with $Al_2(SO_4)_3 \cdot 18H_2O$.

c) Aluminoxane prepared by controlled hydrolysis of $Me_3Al$ with $Al_2(SO_4)_3 \cdot 18H_2O$.

d) Aluminoxane prepared by controlled hydrolysis of $Et_3Al$ with $Al_2(SO_4)_3 \cdot 18H_2O$.

e) Methylaluminoxane and ethylaluminoxane were prepared separately and then mixed in the reactor.

Example 2

The procedure of Example 1 was repeated, but using 3 mmol of aluminoxane and 2 mmol of $(Cp)_2ZrCl2$. The reaction temperature was 115-119°C, pressure was 14.8 bar (200 psig) and time was 0.5 hours. Aluminoxanes prepared individually from $Me_3Al$, $Et_3Al$ and $(i\text{-}Bu)_3Al$ were also used for comparison purposes. The results are shown in Table 2.

Table 2

| Example | Aluminoxane Et:Me:i-Bu | Product Wt. g | Product Distribution $C_4$ | $C_6$ | $C_8$ | $C_{10}$ | $C_{12}$ | $C_{14}$ | $C_{16}$ | $C_{18}$ | $C_{20}^+$ |
|---|---|---|---|---|---|---|---|---|---|---|---|
| 2-1 | 2:1:0[a)] | 12.4 | 26 | 22.3 | 15.4 | 10.1 | 5.7 | 3.5 | 2.7 | 1.0 | 13.3 |
| 2-2 | 1:1:0[b)] | 19.4 | 13.7 | 14.7 | 12.6 | 9.7 | 8.4 | 7.3 | 6.1 | 5.0 | 22.5 |
| 2-3 | 1:1:0[e)] | 19.3 | 13.8 | 16.3 | 14.5 | 10.0 | 8.1 | 6.4 | 5.0 | 3.8 | 22.1 |
| Comparison 2A | 0:1:0[c)] | 9.2 | 3.3 | 4.1 | 5.1 | 5.0 | 5.3 | 5.6 | 5.3 | 5.0 | 61.3 |
| Comparison 2B | 1:0:0[d)] | 2.7 | 32.6 | 27.8 | 11.8 | 5.2 | 2.6 | 1.1 | 0.7 | 0.3 | 17.9 |
| Comparison 2C | 0:1:1[f)] | 10.6 | 3.9 | 4.2 | 6.5 | 6.5 | 5.2 | 5.0 | 4.7 | 4.4 | 59.6 |
| Comparison 2D | 0:0:1[g)] | 2.0 | - | - | - | - | - | - | - | - | - |

a) Aluminoxane prepared by controlled hydrolysis of a 2:1 mixture of $Et_3Al$ and $Me_3Al$ with $Al_2(SO_4)_3.18H_2O$.

b) Aluminoxane prepared by controlled hydrolysis of a 1:1 mixture of $Et_3Al$ and $Me_3Al$ with $Al_2(SO_4)_3.18H_2O$.

c) Aluminoxane prepared by controlled hydrolysis of $Me_3Al$ with $Al_2(SO_4)_3.18H_2O$.

d) Aluminoxane prepared by controlled hydrolysis of $Et_3Al$ with $Al_2(SO_4)_3.18H_2O$.

e) Methylaluminoxane and ethylaluminoxane were prepared separately and then mixed in the reactor.

f) Methylaluminoxane and isobutylaluminoxane were prepared separately and then mixed in the reactor.

g) Aluminoxane prepared by controlled hydrolysis of tri-isobutylaluminium with $Al_2(SO_4)_3.18H_2O$.

## Example 3

Further experiments were carried out according to the method of Example 1, but using different pressures, amounts of zirconocene, aluminoxane and different solvents. 1-Butene was also added (in combination with

ethylene) in one reaction. The aluminoxane had an ethyl to methyl ratio of 2. The reaction time was 0.5 hours. The results are shown in Table 3.

From the results given in Tables 1 to 3, it is clear that the catalyst system according to the invention is surprisingly advantageous for use in the preparation of oligomers of ethylene.

Table 3

| Ex. | $(Cp)_2ZrCl_2$ mmol | Aluminoxane Compound mmol[c)] | Solvent, ml | 1-Butene g | Ethylene[*] bar | Temp °C | Product Wt. g | Product $C_4$-$C_{18}$ % | Product $C_{20}$+ % | Straight-Chain Alpha Olefins % | Vinylidene Olefins % | Internal Olefins % |
|---|---|---|---|---|---|---|---|---|---|---|---|---|
| 3-1 | 3 | 2 | N-heptane 40 ml | 0 | 14.8 | 110-116 | 11.9 | 58 | 42 | 87[a)] | 4[a)] | 9[a)] |
| 3-2 | 3 | 2 | Toluene 40 ml | 0 | 14.8 | 117-120 | 13.2 | 80.3 | 19.7 | 91[b)] | ? | ? |
| 3-3 | 1 | 2 | Toluene 40 ml | 0 | 12.0 | 116-120 | 9.2 | 73.4 | 26.6 | 87.5[b)] | ? | ? |
| 3-4 | 1 | 8 | Toluene 40 ml | 0 | 12.0 | 115-119 | 13.7 | 69 | 31 | 58.4[b)] | ? | ? |
| 3-5 | 2 | 2 | Heptane 20 ml | 7.5 | 14.8 | 115-118 | 8.9 | 81.5 | 18.5 | 70[a)] | 21[a)] | 8[a)] |

a) Determined by $C^{13}$NMR.

b) GC analyses of the $C_{10}$ component allowed for alpha olefin determination but not a distinction between vinylidene olefins and straight chain internal olefins which constituted the remainder.

c) Basis atomic aluminium, that is, one mole of aluminoxane is considered equivalent to one mole of trialkyl aluminium precursor.

* Calculated on the basis that one pound per square inch (psi) is equivalent to 0.0689476 bar.

## Claims

1. A process for preparing oligomers of ethylene, which comprises contacting ethylene, alone or in combination with one or more $C_3$-$C_{10}$ alpha olefins, at an elevated temperature with a catalyst comprising the reaction product of a zirconocene and one or more alkyl aluminoxanes wherein the alkyl is both methyl and ethyl and the ratio of equivalents of methyl to ethyl in the product is in the range of from 4 to 0.25 and wherein the atomic ratio of aluminium to zirconium is in the range of from 0.1 to 100.

2. A process as claimed in claim 1, wherein the oligomerisation reaction is carried out in the absence of oxygen.

3. A process as claimed in claim 1 or 2, wherein the temperature is in the range of from 100 to 130 °C.

4. A process as claimed in any one of claims 1 to 3, wherein the ratio of methyl to ethyl is in the range of from 2 to 0.33.

5. A process as claimed in any one of claims 1 to 4, wherein the zirconocene has the general formula (cyclopentadienyl)$_n$ZrY$_{4-n}$ wherein n is an integer ranging from 1 to 4, Y is selected from hydrogen, a $C_1$-$C_5$ alkyl group, a $C_6$-$C_{20}$ aryl group and halogen, and the cyclopentadienyl groups are optionally substituted with one or more $C_1$-$C_5$ alkyl groups.

6. A process as claimed in claim 5, wherein the zirconocene is selected from bis(cyclopentadienyl)zirconium dichloride, bis(cyclopentadienyl)zirconium methyl chloride, bis(cyclopentadienyl)zirconium dimethyl, bis(methylcyclopentadienyl)zirconium dichloride, bis(methylcyclopentadienyl)zirconium methyl chloride, bis(methylcyclopentadienyl)zirconium dimethyl, bis(pentamethylcyclopentadienyl)zirconium dichloride, bis(pentamethylcyclopentadienyl)zirconium methyl chloride, bis(pentamethylcyclopentadienyl)zirconium dimethyl, bis(n-butyl-cyclopentadienyl)zirconium dichloride, bis(n-butylcyclopentadienyl)zirconium methyl chloride and bis(n-butyl-cyclopentadienyl)zirconium dimethyl.

7. A process as claimed in any one of claims 1 to 6, wherein the atomic ratio of aluminium to zirconium is in the range of from 1 to 20.

8. A process as claimed in any one of claims 1 to 5, wherein the temperature is in the range of from 110 to 125 °C

9. An olefin oligomerization catalyst comprising the reaction product of a zirconocene and one or more alkyl aluminoxanes wherein the alkyl is both methyl and ethyl and the ratio of equivalents of methyl to ethyl in the product is in the range of from 4 to 0.25 and wherein the atomic ratio of aluminium to zirconium is in the range of from 0.1 to 100.

10. A catalyst as claimed in claim 9, wherein the ratio of methyl to ethyl is in the range of from 2 to 0.33.

11. A catalyst as claimed in claim 9 or claim 10, wherein the zirconocene has the general formula (cyclopentadienyl)$_n$ZrY$_{4-n}$ wherein n is an integer ranging from 1 to 4, Y is selected from the group consisting of hydrogen, a $C_1$-$C_5$ alkyl group, a $C_6$-$C_{20}$ aryl group and halogen, and the cyclopentadienyl groups are optionally substituted with one or more $C_1$-$C_5$ alkyl groups.

12. catalyst as claimed in any one of claims 9 to 11, wherein the atomic ratio of aluminium to zirconium is in the range of from 1 to 20.

## Patentansprüche

1. Ein Verfahren zur Herstellung von Oligomeren von Äthylen, welches das Kontaktieren von Athylen allein oder in Kombination mit einem oder mehreren $C_3$-$C_{10}$-Alpha-olefinen bei erhöhter Temperatur mit einem Katalysator, umfassend das Reaktionsprodukt eines Zirkonocens und eines oder mehrerer Alkylaluminoxane, in welchen das Alkyl sowohl Methyl als auch Äthyl ist und das Verhältnis der

Aquivalente von Methyl zu Äthyl im Produkt im Bereich von 4 bis 0,25 liegt und in welchem das Atomverhältnis von Aluminium zu Zirkonium im Bereich von 0,1 bis 100 liegt, umfaßt.

2. Ein Verfahren wie in Anspruch 1 beansprucht, in welchem die Oligomerisierungsreaktion in Abwesenheit von Sauerstoff durchgeführt wird.

3. Ein Verfahren wie in Anspruch 1 oder 2 beansprucht, in welchem die Temperatur im Bereich von 100 bis 130°C liegt.

4. Ein Verfahren wie in einem der Ansprüche 1 bis 3 beansprucht, in welchem das Verhältnis von Methyl zu Athyl im Bereich von 2 bis 0,33 liegt.

5. Ein Verfahren wie in einem der Ansprüche 1 bis 4 beansprucht, in welchem das Zirkonocen die allgemeine Formel (Cyclopentadienyl)$_n$ZrY$_{4-n}$ aufweist, in welcher n eine ganze Zahl im Bereich von 1 bis 4 ist, Y ausgewählt ist aus Wasserstoff, einer $C_1$-$C_5$-Alkylgruppe, einer $C_6$-$C_{20}$-Arylgruppe und Halogen, und die Cyclopentadienylgruppen gegebenenfalls mit einer oder mehreren $C_1$-$C_5$-Alkylgruppen substituiert sind.

6. Ein Verfahren wie in Anspruch 5 beansprucht, in welchem das Zirkonocen ausgewählt ist aus
Bis(cyclopentadienyl)zirkoniumdichlorid,
Bis(cyclopentadienyl)zirkoniummethylchlorid,
Bis(cyclopentadienyl)-zirkoniumdimethyl,
Bis(methylcyclopentadienyl)zirkoniumdichlorid,
Bis(methylcyclopentadienyl)zirkoniummethylchlorid,
Bis(methylcyclopentydienyl)zirkoniumdimethyl,
Bis(pentamethyl-cyclopentadienyl)zirkoniumdichlorid,
Bis(pentamethyl-cyclo-pentadienyl)zirkoniummethylchlorid,
Bis(pentamethylcyclo-pentadienyl) zirkoniumdimethyl, Bis(n-butyl-cyclopentadienyl)-zirkoniumdichlorid, Bis(n-butyl-cyclopentadienyl)zirkoniummethylchlorid und Bis(n-butyl-cyclopentadienyl)zirkoniumdimethyl.

7. Ein Verfahren wie in einem der Ansprüche 1 bis 6 beansprucht, in welchem das Atomverhältnis von Aluminium zu Zirkonium im Bereich von 1 bis 20 liegt.

8. Ein Verfahren wie in einem der Ansprüche 1 bis 5 beansprucht, in welchem die Temperatur im Bereich von 110 bis 125°C liegt.

9. Ein Olefinoligomerisierungskatalysator, umfassend das Reaktionsprodukt eines Zirkonocens und eines oder mehrerer Alkylaluminoxane, in welchen das Alkyl sowohl Methyl als auch Äthyl ist und das Verhältnis der Äquivalente von Methyl zu Äthyl im Produkt im Bereich von 4 bis 0,25 liegt und in welchen das Atomverhältnis von Aluminium zu Zirkonium im Bereich von 0,1 bis 100 liegt.

10. Ein Katalysator wie in Anspruch 9 beansprucht, in welchem das Verhältnis von Methyl zu Äthyl im Bereich von 2 bis 0,33 liegt.

11. Ein Katalysator wie in Anspruch 9 oder 10 beansprucht, in welchem das Zirkonocen die allgemeine Formel (Cyclopentadienyl)$_n$ZrY$_{4-n}$ hat, in welcher n eine ganze Zahl im Bereich von 1 bis 4 ist, Y ausgewählt ist aus der Gruppe, bestehend aus Wasserstoff, einer $C_1$-$C_5$-Alkylgruppe, einer $C_6$-$C_{20}$-Arylgruppe und Halogen, und die Cyclopentadienylgruppen gegebenenfalls mit einer oder mehreren $C_1$-$C_5$-Alkylgruppen substituiert sind.

12. Ein Katalysator wie in einem der Ansprüche 9 bis 11 beansprucht, in welchem das Atomverhältnis von Aluminium zu Zirkonium im Bereich von 1 bis 20 liegt.

## Revendications

1. Procédé de préparation d'oligomères d'éthylène, qui comprend la mise en contact d'éthylène, seul ou en combinaison avec une ou plusieurs alpha-oléfines en $C_3$-$C_{10}$, à une température élevée, avec un catalyseur comprenant le produit de réaction d'un zirconocène et d'un ou plusieurs alkylaluminoxan es dans

lesquels l'alkyle est à la fois le méthyle et l'éthyle et le rapport des équivalents de méthyle à éthyle dans le produit est compris dans l'intervalle allant de 4 à 0,25 et où le rapport atomique de l'aluminium au zirconium est compris dans l'intervalle allant de 0,1 à 100.

2. Procédé selon la revendication 1, dans lequel on effectue la réaction d'oligomérisation en l'absence d'oxygène.

3. Procédé selon la revendication 1 ou 2, dans lequel la température est comprise dans l'intervalle allant de 100 à 130°C.

4. Procédé selon l'une quelconque des revendications 1 à 3, dans lequel le rapport du méthyle à l'éthyle est compris dans l'intervalle allant de 2 à 0,33.

5. Procédé selon l'une quelconque des :revendications 1 à 4, dans lequel le zirconocène possède la formule générale (cyclopentadiényl)$_n$Zr$Y_{4-n}$, dans laquelle n est un nombre entier compris dans l'intervalle allant de 1 à 4, Y est choisi parmi un atome d'hydrogène, un groupe alkyle en $C_1$-$C_5$, un groupe aryle en $C_6$-$C_{20}$ et un atome d'halogène, et les groupes cyclopentadiényle sont éventuellement substitués par un ou plusieurs groupes alkyle en $C_1$-$C_5$.

6. Procédé selon la revendication 5, dans lequel le zirconocène est choisi parmi le dichlorure de bis(cyclopentadiényl)zirconium, le chlorure de bis(cyclopentadiényl)zirconium-méthyle, le bis(cyclopentadiényl)zirconium-diméthyle ; le dichlorure de bis(méthylcyclopentadiényl)zirconium, le chlorure de bis(méthylcyclopentadiényl)zirconium-méthyle, le bis(méthylcyclopentadiényl)zirconium-diméthyle, le dichlorure de bis(pentaméthylcyclopentadiényl)zirconium, le chlorure de bis(pentaméthylcyclopentadiényl)zirconium-méthyle, le bis(pentaméthylcyclopentadiényl)zirconium-diméthyle, le dichlorure de bis(n-butylcyclopentadiényl)zirconium, le chlorure de bis(n-butylcyclopentadiényl)zirconium-méthyle et le bis(n-butylcyclopentadiéhyl)zirconium-diméthyle.

7. Procédé selon l'une quelconque des revendications 1 à 9, dans lequel le rapport atomique de l'aluminium au zirconium est compris dans l'intervalle allant de 1 à 20.

8. Procédé selon l'une quelconque des revendications 1 à 5, dans lequel la température est comprise dans l'intervalle allant de 110 à 125°C.

9. Catalyseur d'oligomérisation d'oléfines qui comprend le produit de réaction d'un zirconocène et d'un ou plusieurs alkylaluminoxanes dans lesquels l'alkyle est à la fois le méthyle et l'éthyle et le rapport des équivalents de méthyle à éthyle dans le produit est compris dans l'intervalle allant de 4 à 0,25 et où le rapport atomique de l'aluminium au zirconium est compris dans l'intervalle allant de 0,1 à 100.

10. Catalyseur selon la revendication 9, dans lequel le rapport du méthyle à l'éthyle est compris dans l'intervalle allant de 2 à 0,33.

11. Catalyseur selon la revendication 9 ou 10, dans lequel le zirconocène possède la formule générale (cyclopentadiényl)$_n$Zr$y_{4-n}$, dans laquelle n est un nombre entier allant de 1 à 4, y est choisi dans le groupe comprenant un atome d'hydrogène, un groupe alkyle en $C_1$-$C_5$, un groupe aryle en $C_6$-$C_{20}$ et un atome d'halogène et les groupes cyclopentadiényle sont éventuellement substitués par un ou plusieurs groupes alkyle en $C_1$-$C_5$.

12. Catalyseur selon l'une quelconque des revendications 9 à 11, dans lequel le rapport atomique de l'aluminium au zirconium est compris dans l'intervalle allant de 1 à 20.